# EUROPEAN PATENT SPECIFICATION

(11) **EP 3 448 799 B1**
(45) Date of publication and mention of the grant of the patent: **19.10.2022**
(21) Application number: 17720439.3
(22) Date of filing: 25.04.2017
(51) Int. Cl.: B67C 7/00, A61L 2/16, B65B 55/10, B65B 57/10, B65B 57/18

(54) **A DEVICE, SYSTEM AND METHOD FOR DETECTING IF A DRYING DEVICE IS PRESENT IN A STERILIZATION SYSTEM**
VORRICHTUNG, SYSTEM UND VERFAHREN ZUM DETEKTIEREN, OB EINE TROCKUNGSVORRICHTUNG IN EINEM STERILISATIONSSYSTEM VORHANDEN IST
DISPOSITIF, PROCÉDÉ ET SYSTÈME POUR DETECTER, SI UN SYSTÈME DE SECHAGE EST PRÉSENT DANS UN SYSTÈME DE STÉRILISATION

(30) Priority: 27.04.2016 SE 1650564
(43) Date of publication of application: 06.03.2019
(73) Proprietor: Tetra Laval Holdings & Finance S.A., 1009 Pully (CH)
(72) Inventor: NILSSON, Stefan, 24441 Kävlinge (SE); NILSSON, Peter Jo, 24545 Staffanstorp (SE); OLSSON, Anders S, 21459 Malmö (SE); LÖFBERG, Henrik, 24161 Löberöd (SE)
(74) Representative: Tetra Pak - Patent Attorneys SE
(86) International application number: PCT/EP2017/059819
(87) International publication number: WO 2017/186730

(56) References cited:
- DE-A1-102004 030 956
- US-A- 3 566 575
- US-A1- 2001 000 558

## Description

### Technical field

The present disclosure relates to the field of packaging. More particularly, to a device, system and method for detecting if a drying device is present in a sterilization system in a filing machine.

### Background

When producing packages for various types of liquid foods it is crucial to assure that the packages are clean from any type of bacteria or similar that could damage the liquid food inserted into the package. Today this is performed by use of hydrogen peroxide (H₂O₂) inserted in various ways into the package such that the H₂O₂ kills all or almost all of the bacteria and assures the cleanliness of the package before any liquid food is inserted in to it. However, H₂O₂ is a strong oxidizer, bleaching agent and disinfectant which has environmental and medical risks associated with it. It is therefore important to keep the use of H₂O₂ at a minimum when disinfecting packages for food liquids and remove as much as possible of the H₂O₂ by e.g. drying the package by blowing out any excessive H₂O₂.

Today this is achieved by use of drying pipes placed at or inserted into the package in a sterilization chamber. However the pipes are not visible or easily visible from outside the sterilization chamber.

Thus, it is desired to have a device, system and method that alert a user that the pipes are not in place and/or correctly placed.

A device according to the preamble of claim 1 is known from US 2001/000558 A1 wherein a control system monitors the air pressure and flow rate in a sterilization tunnel of an application and drying apparatus.

### Summary of the invention

It is an object of the present inventive concept to mitigate, alleviate, or eliminate one or more of the above-identified deficiencies in the art and disadvantages singly or in combination.

According to a first aspect of the disclosure, a detection device according to claim 1 is presented for detecting a presence of at least one drying device for drying H₂O₂ at at least one package and the drying device having a specific pressure signature, comprising, means for detecting a characteristic linked to the specific pressure signature of the at least one drying device, means for determining if the at least one drying device is present based on the detected characteristic.

According to a second aspect ot the disclosure, a system according to claim 4 is presented tor detecting a presence of at least one drying device for drying H₂O₂ at at least one package, comprising, at least one drying device having a specific pressure signature, a detection device, comprising, means for detecting a characteristic linked to the specific pressure signature of the at least one drying device, means for determining if the at least one drying device is present based on the detected characteristic.

According to a third aspect of the disclosure, a method according to claim 7 is presented for detecting a presence of at least one drying device for drying H₂O₂ at at least one package and the drying device having a specific pressure signature, comprising, detecting a characteristic linked to the specific pressure signature of the at least one drying device, determining if the at least one drying device is present based on the detected characteristic.

Further examples of the disclosure are defined in the dependent claims, wherein features for the fourth and subsequent aspects of the disclosure are as for the first to third aspects mutatis mutandis.

Some examples of the disclosure provide for detecting and determining a characteristic linked to a specific pressure signature of at least one drying device and if the drying device is present.

Some examples of the disclosure provide for detecting if a plurality of drying devices is present.

Some examples of the disclosure provide for detecting a threshold target.

Some examples of the disclosure provide for signaling if a drying device is present or not.

Some examples of the disclosure provide for detecting a difference of a frequency which equals a large pressure variation in a drying device.

Some examples of the disclosure provide for a constant pressure at at least one drying device.

Some examples of the disclosure provide for detecting any variation to a pressure in a system.

According to the invention a voltage of a fan that is needed to maintain a constant pressure is detected and analyzed.

Some examples of the disclosure provide for a pre-set and/or variable duration of sampling times.

Generally, all terms used in the claims are to be interpreted according to their ordinary meaning in the technical field, unless explicitly defined otherwise herein. All references to "a/an/the [element, device, component, means, step, etc]" are to be interpreted openly as referring to at least one instance of said element, device, component, means, step, etc., unless explicitly stated otherwise. The steps of any method disclosed herein do not have to be performed in the exact order disclosed, unless explicitly stated.

### Brief description of the drawings

The above, as well as additional objects, features and advantages of the present invention/inventive concept, will be better understood through the following illustrative and non-limiting detailed description of different embodiments of the present invention/inventive concept, with reference to the appended drawings, wherein:
FIG. 1 illustrates a device for detecting if at least one drying pipe is present in a system for drying H₂O₂.
FIG. 2 illustrates a system for detecting if at least one drying pipe is present in a system for drying H₂O₂.
FIG. 3 is a flowchart of a method of detecting if at least one drying pipe is present in a system for drying H₂O₂.

### Detailed description

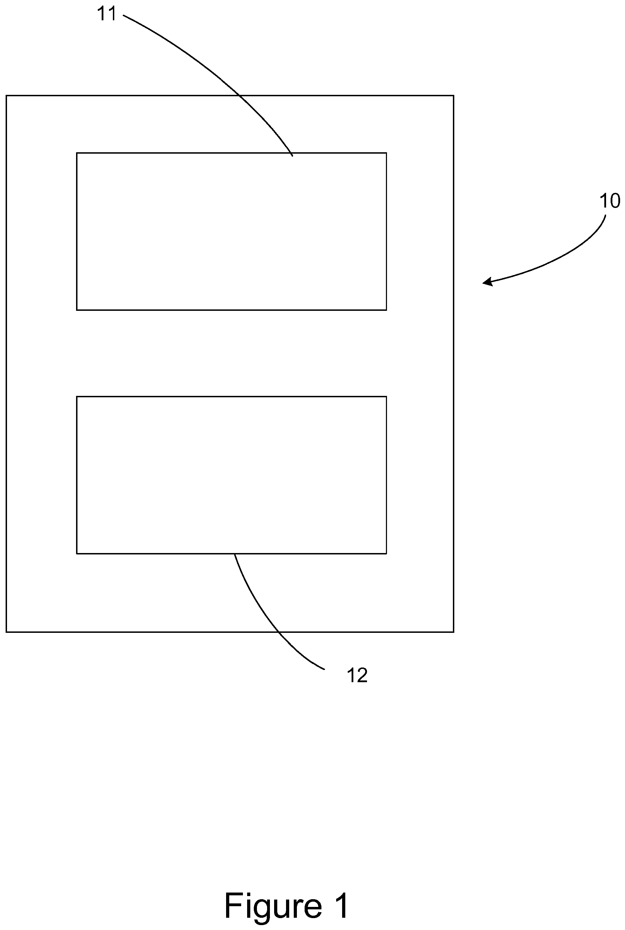

Figure 1 illustrates an example of a detection device 10 for detecting a presence of at least one drying device 20 for drying H₂O₂ at at least one package. The drying device 20 has a specific pressure signature. The detection device 10 comprises means for detecting a characteristic 11 linked to the specific pressure signature of the at least one drying device. The detection device 10 further comprises means for determining 12 if the at least one drying device 20 is present based on the detected characteristic. By detecting and determining the characteristic linked to the specific pressure signature it is possible to detect if the at least one drying device 20 is present.

In an example the detection device 10 is configured to detect if a plurality of drying devices 20 are present. In an example the drying device 20 is a drying pipe.

In an example, the means for detecting the characteristic is a pressure sensor and/or a voltage sensor and/or a current sensor and/or a frequency sensor and/or a current sensor and/or a combination of the above.

In an example, the characteristic is a pressure and/or a voltage and/or a current and/or a frequency.

In an example the means for determining 12 is set to detect a threshold target and which thus signals if a drying device 20 is present or not. In an example the means for determining 12 is set to detect a difference of 4-5Hz which equals a large pressure variation in the drying device 20. In an example the means for determining 12 is a computer, logic circuit, analog circuit and/or digital circuit. In an example the detection device 10 also comprises a memory for temporary storing, or long term storing, of detected characteristics.

In examples, the means for detecting 11 and the means for determining 12 are connected to each other by being wired or wireless.

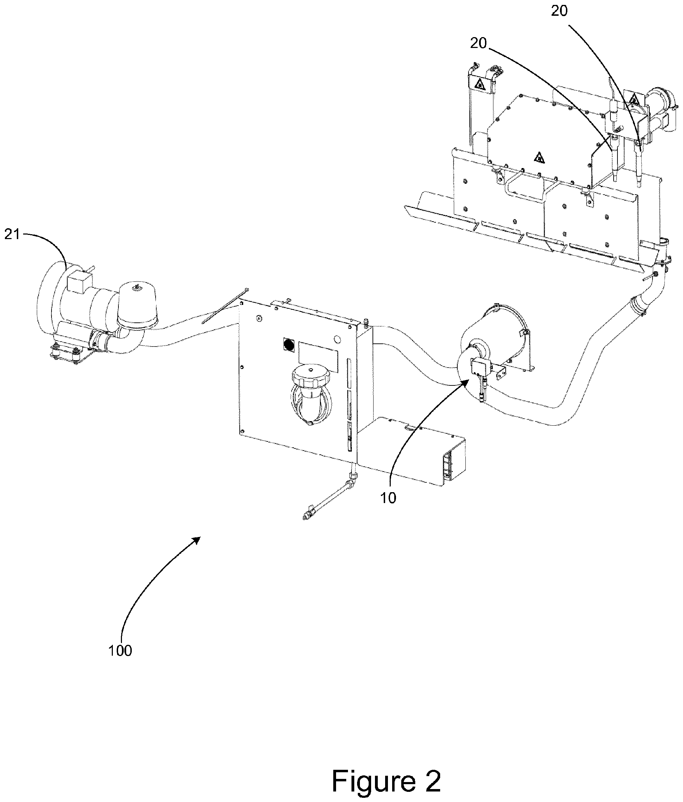

In an example, illustrated in e.g. fig.2 is a system 100 for detecting a presence of at least one drying device 20 for drying H₂O₂ at at least one package. The system 100 comprises at least one drying device 20 having a specific pressure signature. The system 100 also comprises a detection device 10 comprising means for detecting 11 a characteristic linked to the specific pressure signature of the at least one drying device 20 and means for determining 12 if the at least one drying device 20 is present based on the detected characteristic.

The system 100 further comprises a fan 21 for providing a constant pressure at the at least one drying device 20. By having the system 100 comprising the fan that maintains the constant pressure to the at least one drying device 20 any variation to the pressure in the system of e.g. a removal, not correctly placed or not placed at all of the at least one drier 20 will be detectable by the means for detecting 11. The pressure is detected and analyzed by the means for determining 12 if the pressure signature of the at least one drying device 20 is present. In an example, the voltage of the fan 21 that is needed to maintain the constant pressure is detected and analyzed for the characteristic that is linked to the specific pressure signature of the at least one drying device 20 to determine if it is present or not.

In an example, the system 100 comprises one drying device 20 and the fan 21 provides the constant pressure of 80 hectopascal at a frequency of 44-45Hz. In this example the detection device 10 is configured to sample for 30 seconds and evaluate the measured data. Other duration of sampling times is also possible. The detection device 10 is also configured to detect if after and/or during these 30 seconds a frequency above 50 Hz and/or a pressure of 20 hectopascal is present. If so, the detection device 10 signals that the drying device 20 is not present in the system 100.

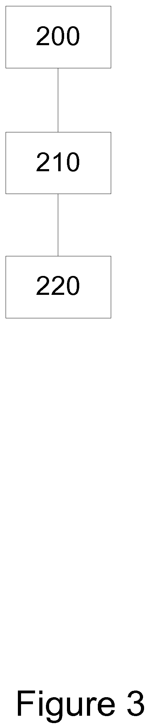

In an example illustrated in figure 3 is a method 200 for detecting a presence of at least one drying device 20 for drying H₂O₂ at at least one package is disclosed. The drying device 20 has a specific pressure signature and the method 200 comprises detecting 210 a characteristic linked to the specific pressure signature of the at least one drying device 20. The method further comprises determining 220 if the at least one drying device 20 is present based on the detected characteristic.

In an example, the detection of the characteristic is by a pressure sensor and/or a voltage sensor and/or a current sensor and/or a frequency sensor and/or a current sensor.

In an example, the characteristic is a pressure and/or a voltage and/or a current and/or a frequency.

In an example, a fan 21 provides a constant pressure to the at least one drying device 20 and the fan speed is controlled by a pressure transmitter, and wherein the determining 220 is based on the characteristic being a pressure detected by the pressure transmitter and/or a frequency corresponding to the fan speed of the fan 21.

Further, the invention has mainly been described with reference to a few embodiments. However, as is readily understood by a person skilled in the art, other embodiments than the ones disclosed above are equally possible within the scope of the invention, as defined by the appended claims.

## Claims

1. A detection device for detecting a presence of at least one drying device (20) for drying H₂O₂ at at least one package and the drying device having a specific pressure signature, comprising,
- means (11) for detecting a characteristic linked to the specific pressure signature of the at least one drying device,
- means (12) for determining if the at least one drying device is present based on the detected characteristic,
**characterized in that**
the means for determining is adapted to detect the voltage of a fan (21) that is needed to maintain a constant pressure at the at least one drying device and the means for determining is adapted to analyse the voltage for the characteristic that is linked to the specific pressure signature of the at least one drying device.

2. A detection device for detecting a presence of at least one drying device according to claim 1, wherein the means for detecting the characteristic comprises a pressure sensor and/or a voltage sensor and/or a current sensor and/or a frequency sensor and/or a current sensor.

3. A detection device for detecting a presence of at least one drying device according to claims 1 or 2, wherein the characteristic is a pressure and/or a voltage and/or a current and/or a frequency.

4. A system (100) for detecting a presence of at least one drying device (20) for drying H₂O₂ at at least one package, comprising,
- at least one drying device having a specific pressure signature,
- a detection device according to claim 1,
the system further comprising a fan (21) for providing a constant pressure at the at least one drying device.

5. A system for detecting a presence of at least one drying device according to claim 4, wherein the means for detecting the characteristic comprises a pressure sensor and/or a voltage sensor and/or a current sensor and/or a frequency sensor and/or a current sensor.

6. A system for detecting a presence of at least one drying device according to any one of claims 4-5, wherein the characteristic is a pressure and/or a voltage and/or a current and/or a frequency.

7. A method for detecting a presence of at least one drying device for drying H₂O₂ at at least one package and the drying device having a specific pressure signature, comprising,
- detecting a characteristic linked to the specific pressure signature of the at least one drying device,
- determining if the at least one drying device is present based on the detected characteristic, wherein determining comprises detecting the voltage of a fan that is needed to maintain the constant pressure and analyzing the voltage for the characteristic that is linked to the specific pressure signature of the at least one drying device.

8. A method according to claim 7, wherein the detecting of the characteristic is by a pressure sensor and/or a voltage sensor and/or a current sensor and/or a frequency sensor and/or a current sensor.

9. A method according to claim 7 or 8, wherein the characteristic is a pressure and/or a voltage and/or a current and/or a frequency.

10. A method according to any one of the preceding claims 7-9,
wherein a fan provides a constant pressure to the at least one drying device and the fan speed is controlled by a pressure transmitter, and wherein the determining is based on the pressure of the pressure transmitter and/or the frequency of the fan speed.

## Patentansprüche

1. Detektionsvorrichtung zum Detektieren eines Vorhandenseins mindestens einer Trocknungsvorrichtung (20) zum Trocknen von H₂O₂ an mindestens einer Verpackung und wobei die Trocknungsvorrichtung eine spezifische Drucksignatur aufweist, umfassend
- Mittel (11) zum Detektieren einer mit der spezifischen Drucksignatur der mindestens einen Trocknungsvorrichtung verbundenen Charakteristik,
- Mittel (12) zum Bestimmen, ob die mindestens eine Trocknungsvorrichtung vorhanden ist, auf Basis der detektierten Charakteristik,
**dadurch gekennzeichnet, dass**
das Mittel zum Bestimmen ausgelegt ist zum Detektieren der Spannung eines Gebläses (21), das benötigt wird, um einen konstanten Druck an der mindestens einen Trocknungseinrichtung aufrechtzuerhalten, und das Mittel zum Bestimmen ausgelegt ist zum Analysieren der Spannung auf die Charakteristik hin, die mit der spezifischen Drucksignatur der mindestens einen Trocknungsvorrichtung verbunden ist.

2. Detektionsvorrichtung zum Detektieren eines Vorhandenseins mindestens einer Trocknungsvorrichtung nach Anspruch 1, wobei das Mittel zum Detektieren der Charakteristik einen Drucksensor und/oder einen Spannungssensor und/oder einen Stromsensor und/oder einen Frequenzsensor und/oder einen Stromsensor umfasst.

3. Detektionsvorrichtung zum Detektieren eines Vorhandenseins mindestens einer Trocknungsvorrichtung nach Ansprüchen 1 oder 2, wobei die Charakteristik ein Druck und/oder eine Spannung und/oder ein Strom und/oder eine Frequenz ist.

4. System (100) zum Detektieren eines Vorhandenseins mindestens einer Trocknungsvorrichtung (20) zum Trocknen von H₂O₂ an mindestens einer Verpackung, umfassend
- mindestens eine Trocknungsvorrichtung mit einer spezifischen Drucksignatur,
- eine Detektionsvorrichtung nach Anspruch 1,
wobei das System ferner ein Gebläse (21) umfasst zum Liefern eines konstanten Drucks an der mindestens einen Trocknungsvorrichtung.

5. System zum Detektieren eines Vorhandenseins mindestens einer Trocknungsvorrichtung nach Anspruch 4, wobei das Mittel zum Detektieren der Charakteristik einen Drucksensor und/oder einen Spannungssensor und/oder einen Stromsensor und/oder einen Frequenzsensor und/oder einen Stromsensor umfasst.

6. System zum Detektieren eines Vorhandenseins der mindestens einen Trocknungsvorrichtung nach einem der Ansprüche 4-5, wobei die Charakteristik ein Druck und/oder eine Spannung und/oder ein Strom und/oder eine Frequenz ist.

7. Verfahren zum Detektieren eines Vorhandenseins mindestens einer Trocknungsvorrichtung zum Trocknen von H₂O₂ an mindestens einer Verpackung und wobei die Trocknungseinrichtung eine spezifische Drucksignatur aufweist, umfassend
- Detektieren einer mit der spezifischen Drucksignatur der mindestens einen Trocknungsvorrichtung verbundenen Charakteristik,
- Bestimmen, ob die mindestens eine Trocknungsvorrichtung vorhanden ist, auf Basis der detektierten Charakteristik, wobei das Bestimmen das Detektieren der Spannung eines Gebläses umfasst, das zum Aufrechterhalten des konstanten Drucks benötigt wird, und Analysieren der Spannung auf die Charakteristik hin, die mit der spezifischen Drucksignatur der mindestens einen Trocknungsvorrichtung verbunden ist.

8. Verfahren nach Anspruch 7, wobei das Detektieren der Charakteristik durch einen Drucksensor und/oder einen Spannungssensor und/oder einen Stromsensor und/oder einen Frequenzsensor und/oder einen Stromsensor erfolgt.

9. Verfahren nach Anspruch 7 oder 8, wobei die Charakteristik ein Druck und/oder eine Spannung und/oder ein Strom und/oder eine Frequenz ist.

10. Verfahren nach einem der vorhergehenden Ansprüche 7-9, wobei ein Gebläse einen konstanten Druck an die mindestens eine Trocknungsvorrichtung liefert und die Gebläsedrehzahl durch einen Druckwandler gesteuert wird, und wobei das Bestimmen auf dem Druck des Druckwandlers und/oder der Frequenz der Gebläsedrehzahl basiert.

## Revendications

1. Dispositif de détection destiné à détecter la présence d'au moins un dispositif de séchage (20) servant à sécher du H₂O₂ au niveau d'au moins un emballage, le dispositif de séchage ayant une signature de pression spécifique, comprenant
- un moyen (11) pour détecter une caractéristique liée à la signature de pression spécifique de l'au moins un dispositif de séchage,
- un moyen (12) pour déterminer si l'au moins un dispositif de séchage est présent sur la base de la caractéristique détectée,
**caractérisé en ce que**
le moyen de détermination est adapté pour détecter la tension d'un ventilateur (21) qui est nécessaire pour maintenir une pression constante au niveau de l'au moins un dispositif de séchage et le moyen de détermination est adapté pour analyser la tension pour la caractéristique qui est liée à la signature de pression spécifique de l'au moins un dispositif de séchage.

2. Dispositif de détection destiné à détecter la présence d'au moins un dispositif de séchage selon la revendication 1, dans lequel le moyen pour détecter la caractéristique comprend un capteur de pression et/ou un capteur de tension et/ou un capteur de courant et/ou un capteur de fréquence et/ou un capteur de courant.

3. Dispositif de détection destiné à détecter la présence d'au moins un dispositif de séchage selon la revendication 1 ou 2, dans lequel la caractéristique est une pression et/ou une tension et/ou un courant et/ou une fréquence.

4. Système (100) destiné à détecter la présence d'au moins un dispositif de séchage (20) servant à sécher du H₂O₂ au niveau d'au moins un emballage, comprenant
- au moins un dispositif de séchage ayant une signature de pression spécifique,
- un dispositif de détection selon la revendication 1,
le système comprenant en outre un ventilateur (21) servant à fournir une pression constante au niveau de l'au moins un dispositif de séchage.

5. Système destiné à détecter la présence d'au moins un dispositif de séchage selon la revendication 4, dans lequel le moyen pour détecter la caractéristique comprend un capteur de pression et/ou un capteur de tension et/ou un capteur de courant et/ou un capteur de fréquence et/ou un capteur de courant.

6. Système destiné à détecter la présence d'au moins un dispositif de séchage selon l'une quelconque des revendications 4 et 5, dans lequel la caractéristique est une pression et/ou une tension et/ou un courant et/ou une fréquence.

7. Procédé destiné à détecter la présence d'au moins un dispositif de séchage servant à sécher du H₂O₂ au niveau d'au moins un emballage, le dispositif de séchage ayant une signature de pression spécifique, comprenant
- la détection d'une caractéristique liée à la signature de pression spécifique de l'au moins un dispositif de séchage,
- la détermination que l'au moins un dispositif de séchage est présent ou non sur la base de la caractéristique détectée, la détermination comprenant la détection de la tension d'un ventilateur qui est nécessaire pour maintenir la pression constante et l'analyse de la tension pour la caractéristique qui est liée à la signature de pression spécifique de l'au moins un dispositif de séchage.

8. Procédé selon la revendication 7, dans lequel la détection de la caractéristique se fait par un capteur de pression et/ou un capteur de tension et/ou un capteur de courant et/ou un capteur de fréquence et/ou un capteur de courant.

9. Procédé selon la revendication 7 ou 8, dans lequel la caractéristique est une pression et/ou une tension et/ou un courant et/ou une fréquence.

10. Procédé selon l'une quelconque des revendications 7 à 9 précédentes, dans lequel un ventilateur fournit une pression constante à l'au moins un dispositif de séchage et la vitesse du ventilateur est régulée par un transmetteur de pression, et dans lequel la détermination est basée sur la pression du transmetteur de pression et/ou la fréquence de la vitesse du ventilateur.
